# EUROPEAN PATENT SPECIFICATION

(11) **EP 2 313 071 B1**
(45) Date of publication and mention of the grant of the patent: **14.06.2017**
(21) Application number: 09798431.4
(22) Date of filing: 18.06.2009
(51) Int. Cl.: A61K 8/37, A61K 8/85, A61Q 1/06

(54) **COMPOSITIONS FOR FORMING LONG WEAR COSMETIC PRODUCTS**
ZUSAMMENSETZUNG ZUR FORMUNG KOSMETISCHER PRODUKTE MIT LANGER ANWENDUNGSZEIT
COMPOSITIONS POUR LA FORMATION DE PRODUITS COSMÉTIQUES À LONGUE TENUE

(30) Priority: 23.06.2008 US 74738 P
(43) Date of publication of application: 27.04.2011
(73) Proprietor: ELC Management LLC, New York, NY 10153 (US)
(72) Inventor: TING-JENULIS, Arlene, G., East Northport NY 11731 (US); BENEDICTO, Cecilia, D., Plainview NY 11803 (US); CASTRO, John, R., Huntington Station NY 11746 (US); STEPNIEWSKI, George, J., Melville NY 11747 (US); MCKERLIE, Mary, Kathleen, Bethpage NY 11714 (US); TABAKMAN, Tatyana, R., NY 11235 (US)
(74) Representative: Hirsch & Associés
(86) International application number: PCT/US2009/047772
(87) International publication number: WO 2010/008753

(56) References cited:
- US-A- 5 985 298
- US-A1- 2004 018 219
- US-A1- 2006 204 460
- US-A1- 2006 257 342
- US-A1- 2008 233 064
- DATABASE GNPD [Online] MINTEL; 1 April 2007 (2007-04-01), "Lipstick", XP002725073, Database accession no. 739436
- DATABASE GNPD [Online] MINTEL; 1 October 2008 (2008-10-01), "Lip Mix", XP002725074, Database accession no. 990568
- DATABASE GNPD [Online] MINTEL; 1 February 2009 (2009-02-01), "Lip Mix", XP002725075, Database accession no. 1069486

## Description

### FIELD OF THE INVENTION

. The present invention relates to a cosmetic composition, more preferably an anhydrous cosmetic composition, which is suitable for forming colored cosmetic products, such as lipsticks, lip glosses, lip liners, foundations, blushes, eye shadows, mascaras, eye liners, and the like. The colored cosmetic products so formed are characterized by exceptionally long wear and a glossy or shiny appearance.

### BACKGROUND OF THE INVENTION

. Color cosmetics have been used for many years to enhancing and highlight certain facial features of the user, such as lips, eyes, cheeks, and the like. In recent years, long wear cosmetic products, such as lipsticks, lip glosses, lip liners, foundations, blushes, eye shadows, mascaras, eye liners, and the like, have gained increasing popularity. These types of products provide longer lasting color and allow fewer applications by the consumers. Further, the long wear cosmetic products are more transfer-resistant and therefore less prone to leaving undesirable marks on cups or clothing.

. One type of cosmetic formulations achieves the long wear characteristic by increasing the amount of colorants. However, cosmetic formulations containing large amounts of colorants tend to become too dry and impart a dry, non-uniform, and "cakey" look to the skin. This is particularly undesirable for lip products, since current fashion trends favor a "wet" or "moist" look of the lips. Although moisturizing and/or conditioning agents can be added to such cosmetic formulations to reduce the dryness, they may undermine the long wear characteristic of the cosmetic formulation and render such formulations less durable on the skin.

. Another type of cosmetic formulations achieves long wear and transfer-resistant characteristics by using large amounts of volatile solvents, which evaporate quickly after application of the cosmetic formulations to the skin and leave a water-proof and transfer-resistant film thereon. However, such cosmetic formulations tend to lose the fresh, glossy, or shiny appearance once the solvents evaporate. Further, the water-proof and transfer-resistant film formed by such cosmetic formulations tends to cause an uncomfortably tight feeling on the lips or the skin.

In MINTEL GNPD, Record ID 739436 has been described a lipstick that delivers color as well as shine. Said lipstick delivers smooth, silky color that lasts and shine and comprises *triisostearoyl* polyglyceryl-3 *dimer dilinoleate.*

. There is therefore a continuing need for improved cosmetic products with not only long wear and transfer-resistant characteristics, but also a long-lasting gloss or shine as well as a comfortable or pleasant feel.

### SUMMARY OF THE INVENTION

. The present invention in one aspect relates to an anhydrous stick-shaped color cosmetic product containing at least one ester of rosin acid, polyglyceryl-2 isostearate/dimer dilinoleate copolymer; and pigments or colorants. The composition contains:
(a) from 0.1 wt% to 50 wt% of an ester of rosin acid;
(b) from 0.1 wt% to 45 wt% of a polyglyceryl-2 isostearate/dimer dilinoleate copolymer; and (c) from 0.1 wt% to 90 wt% of pigments or colorants. Preferably, but not necessarily, the color cosmetic composition of the present invention is anhydrous-based.

. Preferably, the ester of rosin acid is selected from the group consisting of glyceryl rosinate, pentaerythrityl rosinate, silicone rosinate, and combinations thereof. More preferably, the ester of rosin acid is glyceryl rosinate, and most preferably, the glyceryl rosinate is fully or partially hydrogenated. The weight ratio between the ester of rosin acid and the polyglyceryl-2 isostearate/dimer dilinoleate copolymer ranges from about 2:1 to about 1 :5, and more preferably from about 1.5:1 to about 1 :2.5.

. The anhydrous stick-shaped color cosmetic product of the present invention may further contain one or more plasticizers selected from the group consisting of diisostearyl malate, tridecyl trimellitate, polyglyceryl-2 triisostearate, neopentyl glycol diisostearate, and neopentyl glycol dioctanoate. If present such plasticizers can be present in an amount ranging from about 0.1 wt% to about 30 wt%.

. Other aspects and objectives of the present invention will become more apparent from the ensuring description, examples, and claims.

### DETAILED DESCRIPTION OF THE INVENTION AND PREFERRED EMBODIMENTS THEREOF

. The present invention uses a unique combination of two film formers, namely, a rosin acid ester and a polyglyceryl-2 isostearate/dimer dilinoleate copolymer, to form color cosmetic compositions with exceptionally long wear and transfer-resistant characteristics. More importantly, the color cosmetic compositions of the present invention are anhydrous compositions capable of providing an improved glossy or shiny look and a comfortable/pleasant feel that are not available to conventional long wear color cosmetics.

. The first film former employed in the anhydrous stick-shaped color cosmetic product of the present invention is an ester of rosin acid, hereinafter referred to as "rosinate." Rosin acids are acids derived from rosins, which are naturally-occurring resins obtained from pine trees and some other plants, mostly conifers. Specifically, rosins can be produced by heating fresh liquid resin to vaporize the volatile liquid terpene components, and the resulting semi-transparent solid residue contains a mixture of various different resin acids, such as abietic acid, pimaric acid, and their respective isomers. Exemplary rosinates that can be used in the cosmetic compositions of the present invention include hydrogenated or un- hydrogenated esters, such as glyceryl rosinate, pentaerythrityl rosinate, silicone rosinate (as disclosed in US6465673), diethylene glycol rosinate, dimer dilinoleyl hydrogenated rosinate, dipentaerythrityl hexahydroxystearate/hexastearate/ hexarosinate, glyceryl dibehenate/hydrogenated rosinate, glyceryl diisostearate/hydrogenated rosinate, glyceryl trihydrogenated rosinate, glycol rosinate, methyl hydrogenated rosinate, methyl rosinate, pentaerythrityl hydrogenated rosinate, triethylene glycol hydrogenated rosinate, etc. More preferred are: glyceryl rosinate, pentaerythrityl rosinate, silicone rosinate, and combinations thereof, among which glyceryl rosinate is most preferred.

. In a preferred but not necessary embodiment of the present invention, the anhydrous stick-shaped color cosmetic product of the present invention comprise fully or partially hydrogenated glyceryl rosinate. Naturally-occurring rosins contain unsaturated bonds, which may adversely impact the stability of the resulting rosinate upon exposure to heat and oxidation. Hydrogenation of the rosins renders them more stable to heat and oxidation. A particularly preferred rosinate for practice of the present invention is a highly hydrogenated glyceryl rosinate commercially available under the trade name "ENDERE<®> S" from Pinova Solutions at Brunswick, GA.

. The rosinate(s) as described hereinabove may be present in the anhydrous stick-shaped color cosmetic product of the present invention in an amount ranging from about 0.1 wt% to about 50 wt%, preferably from about 1 wt% to about 15 wt%. Note that all weight percentages as provided herein refer to the percentage by total weight of the composition or product.

. The second film former employed in the anhydrous stick-shaped color cosmetic product of the present invention is a polyglyceryl-2 isostearate/dimer dilinoleate copolymer. The polyglyceryl-2 isostearate/dimer dilinoleate copolymer can be produced by a condensation reaction between diglycerine, isostearic acid, and hydrogenated dimer dilinoleic acid. One type of polyglyceryl- 2 isostearate/dimer dilinoleate copolymer that is preferably suitable for use in the cosmetic compositions of the present invention is characterized by a number average molecular weight ranging from about 4000 to about 5000 g/mol and is commercially available under the trade name "HAILUCENT ISDA" from Kokyu Alcohol Kogyo Co. in Japan.

. The polyglyceryl-2 isostearate/dimer dilinoleate copolymer as described hereinabove can be present in the cosmetic compositions of the present invention in an amount ranging from about 0.1 wt% to about 45 wt%, preferably from about 1 wt% to about 15 wt%. Note that it is particularly preferred to maintain the weight ratio between the rosinate and the polyglyceryl-2 isostearate/dimer dilinoleate copolymer within a range of from about 2:1 to about 1 :5 in the cosmetic compositions of the present invention. Although not wishing to be bound by any particular theory, it is believed by the inventors that the combined use of the rosinate and the polyglyceryl-2 isostearate/dimer dilinoleate copolymer within such a weight ratio specific range is important for forming a flexible film with sufficient adhesion to the skin and sufficient shine.

. For forming color cosmetic products, the anhydrous stick-shaped color cosmetic product of the present invention further comprise one or more organic or inorganic pigments or colorants. Examples of suitable inorganic pigments include, but are not limited to: iron oxides (yellow, red, brown or black), titanium dioxide (white), zinc oxide, chrome oxide (green), chrome hydrate (green), ultramarines, manganese violet, ferric ferrocyanide, carmine 40, ferric ammonium ferrocyanide, or combinations thereof. Interference pigments, which are thin platelike layered particles having a high refractive index, and which, at a certain thickness, produce interference colors, resulting from the interference of typically two, but occasionally more light reflections from different layers of the plate, can also be added to provide a pearlescence to the product. Suitable organic pigments for use in the composition of the present invention include, but are not limited to: natural colorants, synthetic monomeric and polymeric colorants, such as phthalocyanine blue and green pigment, diarylide yellow and orange pigments, and azo-type red and yellow pigments such as toluidine red, litho red, naphthol red and brown pigments. Also useful are lakes, which are pigments formed by the precipitation and absorption of organic dyes on an insoluble base, such as alumina, barium, or calcium hydrates. Particularly preferred lakes are primary FD&C or D&C Lakes and blends thereof. Stains, such as bromo dyes and fluorescein dyes can also be employed. The cosmetic compositions of the present invention may also contain one or more types of cosmetically acceptable glitter, i.e., particles of transparent or colored, solid organic materials, such as poly (ethylene terephthalate), polymethacrylate, and poly (vinylbutyral), particles of metal, or particles of metal coated film or paper. The total amount of pigments or colorants in the cosmetic composition of the present invention may range from about 0.1 wt% to about 90 wt%, more preferably from about 2 wt% to about 80 wt%.

. The anhydrous stick-shaped color cosmetic product of the present invention can also contain inorganic powders, such as soft focus powders, or plate-like non-spherical powders such as bismuth oxychloride, boron nitride, barium sulfate, mica, sericite, muscovite, synthetic mica, titanium oxide coated mica, titanium oxide-coated bismuth oxychloride, titanium oxide coated talc, platelet iron oxides, metal powders such as aluminum, lauroyl lysine and platelet talc. Amounts of such inorganic powders are not critical, but if used, typically will be used in an amount of about 0.5 to about 5%.

. In order to improve the spreadability and feel of the anhydrous stick-shaped color cosmetic product of the present invention on the skin, it is desirable to incorporate one or more plasticizers into such compositions which plasticizes the film formed by the rosinate ester. Examples of suitable plasticizers include C2-30 mono-, di-, or tricarboxylic acid esters of mono-, di-, or polyhydric C2-20 alcohols. Examples of carboxylic acids include malic, trimellitic, isostearic, stearic, palmitic, octanoic, pentanoic, behenic acids and so on. Examples of alcohols include polyhydric alcohols such as glycerin or polyglycerin with 2 to 10 repeating groups; neopentyl glycol, and so on. Preferred plasticizers include, but are not limited to: diisostearyl malate, tridecyl trimellitate, polyglyceryl-2 triisostearate, neopentyl glycol diisostearate, neopentyl glycol dioctanoate, and other similar esters with medium to high viscosity. Suggested amount of plasticizer as used in the cosmetic compositions of the present invention ranges from about 0.1 wt% to about 30 wt%, more preferably from about 5 wt% to about 25 wt%.

. The anhydrous stick-shaped color cosmetic product of the present invention is particularly suitable for forming products, such as lipsticks, lip liners, foundation sticks, blush sticks, body makeup sticks, eye shadow sticks, eye liners, and the like. In order to increase the shape retention properties of cosmetic compositions, the compositions of the present invention preferably include one or more structuring agents, such as natural waxes or synthetic waxes. If present suggested ranges are from about 0.1 to 70%, preferably from about 0.5 to 50%. Suitable natural waxes that can be used in the present invention include, but are not limited to: candelilla, carnauba waxes, beeswax, spermaceti, carnauba, baysberry, montan, ozokerite, ceresin, paraffin, jojoba wax, castor wax, buckwheat wax, sugar cane wax, or any hydrogenated vegetable oils. Suitable synthetic waxes that can be used in the present invention include, but are not limited to: Fisher-Tropsch waxes such as synthetic wax or polyethylene, silicone waxes (e.g., DC 2503 from Dow Corning), microcrystalline waxes, polyethylene waxes, polystyrene waxes, polypropylene waxes, polyurethane waxes, sugar/saccharide/polysaccharide derivatives, and the like. Also suitable are fatty alcohols that are solid or semi-solid at room temperature including stearyl alcohol, cetyl alcohol, oleyl alcohol, isocetyl alcohol, and so on. Since a high wax concentration may destroy the gloss or shine of the cosmetic composition, it is preferred to keep the total amount of waxes in the composition of the present invention below 10%, more preferably below 8%.

. The anhydrous stick-shaped color cosmetic product of the present invention may contain one or more skin care additives, which are agents that provide benefits to the skin, rather than merely improving the physical or aesthetic characteristics of such composition. If present, such skin care actives may range from about 0.01 to 50%, preferably from about 0.05 to 35% by weight of the total composition. Exemplary skin care additives that can be used in the cosmetic compositions of the present invention include, but are not limited to: sunscreen agents, self-tanning agents, anti-aging agents, anti-wrinkle agents, anti-acne agents (e.g., resorcinol, salicylic acid, and the like), enzyme-inhibiting agents, collagen-stimulating agents, agents for the eradication of age spots and keratoses, analgesics, anesthetics, antimicrobials (e.g., antibacterials, antiyeast agents, antifungal agents, and antiviral agents), antidandruff agents, antidermatitis agents, antipruritic agents, antiemetics, anti-inflammatory agents, antihyperkeratolytic agents, antiperspirants, antipsoriatic agents, antiseborrheic agents, antihistamine agents, skin lightening agents, depigmenting agents, skin soothing/healing agents (e.g., aloe vera extract, allantoin, and the like), corticosteroids, hormones, antioxidants, proteins or peptides, vitamins and derivatives thereof (e.g., vitamin A, vitamin E, vitamin B3, vitamin B5, and the like), exfoliants, retinoids (e.g., retinoic acid and retinol), farnesol, bisabolol, phytantriol, glycerol, urea, guanidine (e.g., amino guanidine), clotrimazole, ketoconazole, miconozole, griseofulvin, hydroxyzine, diphenhydramine, pramoxine, lidocaine, procaine, mepivacaine, monobenzone, erythromycin, tetracycline, clindamycin, meclocyline, minocycline, hydroquinone, naproxen, ibuprofen, theophylline, cromolyn, albuterol, topical steroids (e.g., hydrocortisone, hydrocortisone 21 -acetate, hydrocortisone 17-valerate, and hydrocortisone 17-butyrate), betamethasone valerate, betamethasone diproprionate, benzoyl peroxide, crotamiton, propranolol, promethazine, and mixtures or derivatives thereof. In a preferred, but not necessary embodiment of the present invention, the topical composition comprises one or more skin care actives selected from the group consisting of sunscreen agents, self-tanning agents, anti-aging agents, anti-wrinkle agents, anti-acne agents, antimicrobials, anti- inflammatory agents, skin-lightening agents, antioxidants, proteins or peptides, vitamins and derivatives thereof, exfoliants, and mixtures thereof.

. The anhydrous stick-shaped color cosmetic product of the present invention preferably comprises from about 0.1 wt% to about 85 wt%, more preferably from 5 wt% to about 85 wt%, and most preferably from about 10 wt% to about 75 wt% of a cosmetically acceptable carrier, for solubilizing or dispersing the above-described components or ingredients. Such cosmetically acceptable carrier preferably comprises one or more oils or other liquid materials. More preferably, the cosmetically acceptable carrier is an anhydrous liquid. The term "anhydrous" as used herein means that water is not intentionally added to the composition.

. A variety of volatile oils, nonvolatile oils, and mixtures thereof can readily be used as the cosmetically acceptable carrier in the present invention. If present, suggested ranges are from about 0.1 to 80%. For example, the cosmetically acceptable carrier may include volatile or non-volatile silicones, such as cyclomethicone; methyl trimethicone; octamethyltrisiloxane; decamethyltetrasiloxane; dodecamethylpentasiloxane; dimethicone; phenyl trimethicone trimethylsiloxyphenyl dimethicone; phenyl dimethicone; cetyl dimethicone; dimethicone copolyol, cetyl dimethicone copolyol; glycerolated silicones such as lauryl PEG-9 polydimethylsiloxyethyl dimethicone; or mixtures thereof. For another example, the cosmetically acceptable carrier may include various volatile or non- volatile hydrocarbons, such as pentane, hexane, heptane, decane, dodecane, isododecane, tetradecane, tridecane, Cg-C20 isoparaffins, monomeric or polymeric olefins or alpha olefins, such as polyisobutene, polydecene, polybutene, or hydrogenated derivatives thereof. For a still further example, the cosmetically acceptable carrier may include esters include mono-, di-, or triesters of C4_3o fatty acids and mono-, di-, or polyhydric Ci_2o alcohols, such as fatty acid (e.g., stearyl, behenyl, and isostearyl) esters of glycerin, or fatty acid esters of alpha hydroxyl acids such as citric, malic, or lactic acids and the like. Such esters include pentaerythrtityl tetraisostearate, bis- behenyl/isostearyl/phytostearyl dimer dilinoleyl dimer dilinoleate glyceryl monostearate, isopropyl isostearate, stearic acid, isostearic acid, isocetyl stearate, castor isostearate succinate, isopropyl stearate, butyl stearate, isopropyl laurate, hexyl laurate, decyl oleate, isobutyl palmitate, cetyl palmitate, isopropyl palmitate, palmitic acid, dimethylpolysiloxane, glyceryl monoricinoleate, di-n-butyl sebacate, isopropyl myristate, butyl myristate, myristyl myristate, isopropyl linoleate, lauryl lactate, myristyl lactate, polyethylene glycol, triethylene glycol, lanoline, acetylated lanolin, sesame oil, coconut oil, arrachis oil, castor oil, mink oil, mineral oil, pomegranate sterols, and petroleum. Among the above-listed anhydrous liquid carriers, a volatile paraffinic hydrocarbon such as isododecane is particularly preferred.

. The cosmetically acceptable carrier may comprise one or more humectants. If present, they may range from about 0.1 to 20% by weight of the total composition and include polyhydric alcohols including glycerol, Ci_4 alkylene glycols such as butylene, propylene, ethylene glycol, glycerin, and the like, polyalkylene glycols, and alkylene polyols and mixtures thereof, hyaluronic acid, urea, sorbitol, sodium 2-pyrrolidone-5-carboxylate, soluble collagen, dibutylphthalate and gelatin.

. A variety of water soluble preservatives can be added to the anhydrous stick-shaped color cosmetic product of the present invention to provide a prolonged shelf life. Suitable preservatives include, but are not limited to: potassium sorbate, imidazolidinyl urea, p-hydroxy benzoate, esters of p- hydroxybenzoic acid, various parabens (as disclosed in the 12th Edition of CTFA's International Cosmetic Ingredient Dictionary and Handbook), ethylhexylglycerin, caprylyl glycol/phenoxyethanol/hexylene glycol, and the like. Other preservatives suitable for use in the cosmetic compositions of the present invention are disclosed in the 12th Edition of CTFA's International Cosmetic Ingredient Dictionary and Handbook.

. The cosmetic composition of the present invention may optionally comprise a fragrance in an amount sufficient to make the composition more appealing to the consumer. Preferably, the fragrance is in the amount of from about 0.001% to about 10% by total weight of the composition.

. Although the most preferred embodiment of the invention is an anhydrous, oilbased composition, it is possible to formulate the compositions of the invention as an emulsion, such as a water-in-oil emulsion, an oil-in-water emulsion, and the like, while the above-described ingredients can be used to form the oil phase of such emulsion. The water phase of the emulsion can also contain water-soluble actives. Furthermore, although a particularly preferred use of the cosmetic compositions of the present invention is in forming lipsticks, it may also be used for forming other types of stick-shaped cosmetic products, such as, for example, foundation sticks, blush sticks, eye shadow sticks, eyeliners, body make-up sticks, and the like. The cosmetic compositions of the present invention can also be used to form non-stick-shaped cosmetic products, as described hereinabove.

## Claims

1. An anhydrous stick-shaped color cosmetic product comprising:
(a) from 0.1 w% to 50 w% of an ester of rosin acid;
(b) from 0.1 w% to 45 w% of polyglyceryl-2 isostearate/dimer dilinoleate copolymer; and
(c) from 0.1 wt% to 90 wt% of pigments or colorants;
wherein the weight ratio between the ester of rosin acid and the polyglyceryl-2 isostearate/dimer dilinoleate copolymer ranges from about 2:1 to about 1:5.

2. The anhydrous stick-shaped color cosmetic product of claim 1, wherein the ester of rosin acid is selected from the group consisting of glyceryl rosinate, pentaerythrityl rosinate, silicone rosinate, diethylene glycol rosinate, dimer dilinoleyl hydrogenated rosinate, dipentaerythrityl hexahydroxystearate/hexastearate/hexarosinate, glyceryl dibehenate/hydrogenated rosinate, glyceryl diisostearate/hydrogenated rosinate, glyceryl trihydrogenated rosinate, glycol rosinate, methyl hydrogenated rosinate, methyl rosinate, pentaerythrityl hydrogenated rosinate, triethylene glycol hydrogenated rosinate; and mixtures thereof.

3. The anhydrous stick-shaped color cosmetic product of claim 2, wherein the ester of rosin acid is selected from the group consisting of glyceryl rosinate, pentaerythrityl rosinate, silicone rosinate, and combinations thereof.

4. The anhydrous stick-shaped color cosmetic product of claim 3, wherein the ester of rosin acid is glyceryl rosinate.

5. The anhydrous stick-shaped color cosmetic product of claim 4, wherein the glyceryl rosinate is partially or fully hydrogenated.

6. The anhydrous stick-shaped color cosmetic product according to any one of the preceding claims, wherein the ester of rosin acid is present in an amount ranging from 1 wt% to 15 wt%.

7. The anhydrous stick-shaped color cosmetic product according to any one of the preceding claims, wherein the polyglyceryl-2 isostearate/dimer dilinoleate copolymer is present in an amount ranging from 1 wt% to 10 wt%.

8. The anhydrous stick-shaped color cosmetic product according to any one of the preceding claims, further comprising one or more plasticizers.

9. The anhydrous stick-shaped color cosmetic product of claim 8, wherein the plasticizers are selected from the group consisting of C₂₋₃₀ mono-, di-, or tricarboxylic acid esters of mono-, di-, or polyhydric C₂₋₂₀ alcohols.

10. The anhydrous stick-shaped color cosmetic product of claim 9, wherein said plasticizers are selected from the group consisting of diisostearyl malate, tridecyl trimellitate, polyglyceryl-2 triisostearate, neopentyl glycol diisostearate, and neopentyl glycol dioctanoate.

11. The anhydrous stick-shaped color cosmetic product of claim 8, wherein said plasticizers are present in an amount ranging from 0.1 wt% to 30 wt%.

12. The anhydrous stick-shaped color cosmetic product according to any one of the preceding claims, further comprising at least one silicone or hydrocarbon oil or mixtures thereof.

## Patentansprüche

1. Ein wasserfreies stiftförmiges Farbkosmetikprodukt, das Folgendes umfasst:
(a) 0,1 Gew.-% bis 50 Gew.-% eines Rosinsäureesters;
(b) 0,1 Gew.-% bis 45 Gew.-% Polyglyceryl-2 Isostearat/ Dilinoleat Dimer Copolymer; und
(c) 0,1 Gew.-% bis 90 Gew.-% Pigmente oder Farbstoffe;
wobei das Gewichtsverhältnis zwischen dem Rosinsäureester und dem Polyglyceryl-2 Isostearat/ Dilinoleat Dimer Copolymer im Bereich von etwa 2:1 bis etwa 1:5 liegt.

2. Das wasserfreie stiftförmige Farbkosmetikprodukt nach Anspruch 1, wobei der Rosinsäureester aus der Gruppe bestehend aus Glyceryl Rosinat, Pentaerythrityl Rosinat, Silicon Rosinat, Diethylen-Glycol Rosinat, Dilinoleyl Dimer hydriertem Rosinat, Dipentaerythrityl Hexahydroxystearat/Hexastearat/Hexarosinat, Glyceryl Dibehenat/hydriertem Rosinat, Glyceryl Diisostearat/ hydriertem Rosinat, Glyceryl tri-hydriertem Rosinat, Glycol Rosinat, Methyl hydriertem Rosinat, Methyl Rosinat, Pentaerythrityl hydriertem Rosinat, Triethylen Glycol hydriertem Rosinat; und Gemischen davon ausgewählt ist.

3. Das wasserfreie stiftförmige Farbkosmetikprodukt nach Anspruch 2, wobei der Rosinsäureester aus der Gruppe bestehend aus Glyceryl Rosinat, Pentaerythrityl Rosinat, Silicon Rosinat und Kombinationen davon ausgewählt ist.

4. Das wasserfreie stiftförmige Farbkosmetikprodukt nach Anspruch 3, wobei der Rosinsäureester Glyceryl Rosinat ist.

5. Das wasserfreie stiftförmige Farbkosmetikprodukt nach Anspruch 4, wobei das Glyceryl Rosinat teilweise oder vollständig hydriert ist.

6. Das wasserfreie stiftförmige Farbkosmetikprodukt nach einem jeden der vorhergehenden Ansprüche, wobei der Rosinsäureester in einer Menge im Bereich von 1 Gew.-% bis 15 Gew.-% vorhanden ist.

7. Das wasserfreie stiftförmige Farbkosmetikprodukt nach einem jeden der vorhergehenden Ansprüche, wobei das Polyglyceryl-2 Isostearat/Dilinoleat Dimer Copolymer in einer Menge im Bereich von 1 Gew.-% bis 10 Gew.-% vorhanden ist.

8. Das wasserfreie stiftförmige Farbkosmetikprodukt nach einem jeden der vorhergehenden Ansprüche, das ferner einen oder mehrere Weichmacher umfasst.

9. Das wasserfreie stiftförmige Farbkosmetikprodukt nach Anspruch 8, wobei die Weichmacher aus der Gruppe bestehend aus C₂₋₃₀-Mono-, Di- oder Tricarbonsäureestern ein-, zwei- oder mehrwertiger C₂₋₂₀-Alkohole ausgewählt sind.

10. Das wasserfreie stiftförmige Farbkosmetikprodukt nach Anspruch 9, wobei die Weichmacher aus der Gruppe bestehend aus Diisostearyl Malat, Tridecyl Trimellitat, Polyglyceryl-2 Triisostearat, Neopentyl Glycol Diisostearat und Neopentyl Glycol Dioctanoat ausgewählt sind.

11. Das wasserfreie stiftförmige Farbkosmetikprodukt nach Anspruch 8, wobei die Weichmacher in einer Menge im Bereich von 0,1 Gew.-% bis 30 Gew.-% vorhanden sind.

12. Das wasserfreie stiftförmige Farbkosmetikprodukt nach einem jeden der vorhergehenden Ansprüche, das ferner mindestens ein Silicon- oder Kohlenwasserstofföl oder Gemische davon umfasst.

## Revendications

1. Produit cosmétique coloré anhydre sous forme de stick comprenant :
(a) de 0,1 à 50 % en poids d'un ester d'acide de colophane ;
(b) de 0,1 à 45 % en poids d'un copolymère de 2-isostéarate de polyglycéryle/dimère dilinoléate ; et
(c) de 0,1 à 90 % en poids de pigments ou de colorants ;
dans lequel le rapport en poids entre l'ester d'acide de colophane et le copolymère de 2-isostéarate de polyglycéryle/dimère dilinoléate est dans l'intervalle d'environ 2:1 à environ 1:5.

2. Produit cosmétique coloré anhydre sous forme de stick selon la revendication 1, dans lequel l'ester d'acide de colophane est choisi dans le groupe constitué par le rosinate de glycéryle, le rosinate de pentaérythrityle, le rosinate de silicone, le rosinate de diéthylène glycol, le rosinate hydrogéné de dimère dilinoléyle, l'hexahydroxystéarate/hexastéarate/hexarosinate de dipentaérythrityle, le dibéhénate de glycéryle/rosinate hydrogéné, le diisostéarate de glycéryle/rosinate hydrogéné, le rosinate de glycéryle trihydrogéné, le rosinate de glycol, le rosinate de méthyle hydrogéné, le rosinate de méthyle, le rosinate de pentaérythrityle hydrogéné, le rosinate de triéthylène glycol hydrogéné; et leurs mélanges.

3. Produit cosmétique coloré anhydre sous forme de stick selon la revendication 2, dans lequel l'ester d'acide de colophane est choisi dans le groupe constitué par le rosinate de glycéryle, le rosinate de pentaérythrityle, le rosinate de silicone, et leurs combinaisons.

4. Produit cosmétique coloré anhydre sous forme de stick selon la revendication 3, dans lequel l'ester d'acide de colophane est le rosinate de glycéryle.

5. Produit cosmétique coloré anhydre sous forme de stick selon la revendication 4, dans lequel le rosinate de glycéryle est partiellement ou entièrement hydrogéné.

6. Produit cosmétique coloré anhydre sous forme de stick selon l'une quelconque des revendications précédentes, dans lequel l'ester d'acide de colophane est présent dans une quantité allant de 1 à 15 % en poids.

7. Produit cosmétique coloré anhydre sous forme de stick selon l'une quelconque des revendications précédentes, dans lequel le copolymère de 2-isostéarate de polyglycéryle/dimère dilinoléate est présent dans une quantité allant de 1 à 10 % en poids.

8. Produit cosmétique coloré anhydre sous forme de stick selon l'une quelconque des revendications précédentes, comprenant en outre un ou plusieurs plastifiants.

9. Produit cosmétique coloré anhydre sous forme de stick selon la revendication 8, dans lequel les plastifiants sont choisis dans le groupe constitué par les esters d'acides mono-, di-, ou tricarboxyliques en C₂₋₃₀ d'alcools monohydriques, dihydriques, ou polyhydriques en C₂₋₂₀.

10. Produit cosmétique coloré anhydre sous forme de stick selon la revendication 9, dans lequel ledits plastifiants sont choisis dans le groupe constitué par le malate de diisostéaryle, le trimellitate de tridécyle, le 2-triisostéarate de polyglycéryle, le diisostéarate de néopentylglycol, et le dioctanoate de néopentylglycol.

11. Produit cosmétique coloré anhydre sous forme de stick selon la revendication 8, dans lequel lesdits plastifiants sont présents en une quantité dans la plage de 0,1 à 30 % en poids.

12. Produit cosmétique coloré anhydre sous forme de stick selon l'une quelconque des revendications précédentes, comprenant en outre au moins une huile de silicone ou une huile hydrocarbonée ou leurs mélanges.
